# EUROPEAN PATENT SPECIFICATION

(11) **EP 3 111 995 B1**
(45) Date of publication and mention of the grant of the patent: **08.04.2020**
(21) Application number: 16176302.4
(22) Date of filing: 27.06.2016
(51) Int. Cl.: A61Q 9/02, A61K 8/86, A61K 8/894, B26B 21/44, C10M 107/50

(54) **LIQUID COMPOSITIONS FOR HAIR REMOVAL DEVICES**
FLÜSSIGE ZUSAMMENSETZUNGEN FÜR HAARENTFERNUNGSVORRICHTUNGEN
COMPOSITIONS LIQUIDES POUR DISPOSITIFS D'ÉLIMINATION DE POILS

(30) Priority: 30.06.2015 EP 15174445
(43) Date of publication of application: 04.01.2017
(73) Proprietor: The Gillette Company LLC, Boston, MA 02127 (US)
(72) Inventor: Bradford, Valerie Jean, Boston (US); Stephens, Alison Fiona, Egham, Surrey TW20 9NW (GB); Williamson, Martin Stephen, Egham, Surrey TW20 9NW (GB)
(74) Representative: P&G Patent Germany

(56) References cited:
- WO-A1-91/07943
- DE-A1-102013 219 980
- GB-A- 1 299 089

## Description

### FIELD OF THE INVENTION

The invention relates to liquid compositions comprising a silicone polyether bock copolymer exhibiting improved lubricating properties and their use in hair removal devices.

### BACKGROUND OF THE INVENTION

The use of solid shaving aids or liquid compositions with hair removal devices before or during the shaving process to provide lubrication benefits is known in the art. Lubricating members are typically located on the razor cartridge and release the actives upon contact with water during the shaving process. Alternatively lubrication may be delivered by the use of liquid or foaming shaving compositions applied before the shaving process or contained within the razor handle and which are dispensed during shaving by activation by the user. Foaming shaving compositions are described for example in WO91/07943, DE10201321980 and GB1299089.

However, since the introduction of polyethylene oxide (polyox) as a shaving lubricant as described in the art above for example, little development has been made in the field, even though polyethylene oxide polymers are not without limitations. For example, utilizing polyethylene oxide polymers having low molecular weights or high molecular weights may provide a means to improve lubrication, but may also result in trade off with regard to residue and or stringiness or other aspects of the aqueous solution typically formed in-use. For example, the resultant viscosity in aqueous solution may also increase, leading to negatively perceived attributes, for example concerning the feeling of the shave for the user, particularly in respect of the lubricant. The prior art does also describe the use of combinations of high and low molecular weight polyethylene oxide polymers in order to balance these performance attributes. Nevertheless, such combinations are also limited in their ability to improve performance and or suffer from other negative performance attributes.

The solid shaving aid art further describes the incorporation of additional materials such as oils to further improve the lubrication performance as described in for example US6442839, US2007/0110703 US2009/0223057, and US2008/0060201. However such solid shaving aids exhibit a reduction of the swelling and solubility of water soluble shaving aid contained in the water insoluble polymer matrix. The ability of the shaving aid to swell in contact with water is however believed to be the key mechanism by which the lubrication benefit is delivered to the skin. Hence this is not desirable, as it will negatively impact the overall performance.

Several different composition dispensing razors are known in the art as described in e.g., U.S. 7,007,389, US6,308,413, US4,753,006, US4,635,361, US6,986,207, US5,855,066 and US4,129,942. Such dispensing razors have been described as being capable of dispensing various types of shaving related preparations, including clear or translucent shaving gels or lotions. Compositions intended for liquid dispensing in addition to providing lubrication also need to ensure the desired viscosity. For example a less viscous formulation may be desirable in certain instances, such as where the formulator wants the composition to dispense in a discrete area but quickly spread to contact and/or coat a large surface, such as the shaving head and cutters. It can also be desirable, however, for the product to be sufficiently thick so it will not run off or otherwise be pushed away from the portion of skin desired for treatment. Many different types of thickeners and viscosity modifying agents can impact the viscosity and rheology of the composition. Many of these ingredients, however, also impact other characteristics of the composition when added, such as making the composition stringy or tacky, or making the composition cloudy or opaque which may not be desirable in certain embodiments.

One other class of ingredients which is known to provide lubrication benefits includes surfactants, particularly soaps. Many of these surfactants, however, are capable of causing undesirable skin irritation during and following use in certain instances, particularly in hard water areas. This can be particularly relevant where users do not wash off the composition from skin following the hair removal process, which may exacerbate skin irritation.

Consequently, there is still a need to provide a liquid composition to be utilized before or during shaving exhibiting improved lubricating properties which can be readily manufactured without impacting performance and which can be readily applied and left on the skin without causing negative skin impacts such as irritation or stringiness.

Silicone polyether block copolymers have been described in the literature to provide a number of benefits such as foaming, defoaming, wetting, deaceration and lubricity. However it has been now been surprisingly found that the selection of silicone block copolymers having from 20% to 90% by weight of polypropylene, from 1% to 50% by weight of polyethylene oxide and from 1% to 20% by weight of silicone unexpectedly provide improved lubrication whilst ensuring the required level of water dispersion without impacting stringiness or residue or skin irritation. Moreover, the use of such silicone block copolymers can be further improved in combination with other lubricants such as copolymers of polyethylene oxide and polypropylene oxide.

### SUMMARY OF THE INVENTION

One aspect of the invention relates to a composition dispensing hair removal device, said device containing a liquid composition comprising from 0.1% to 60% by weight of a silicone polyether block copolymer, wherein said silicone polyether block copolymer comprises from 1 to 50%, by weight of polyethylene oxide, from 20% to 90% by weight of polypropylene oxide and from 1% to 20% by weight of silicone, wherein said silicone polyether block copolymer has a molecular weight of from 10000 to 15000.

Another aspect of the invention relates to a personal care composition comprising from 40% to 95%, preferably 60% to 95% by weight of water, 1% to 6% by weight of a volatile post foaming agent and from 0.1% to 60% by weight of a silicone polyether block copolymer wherein said silicone polyether block copolymer comprises from 1 to 50%, by weight of polyethylene oxide, from 20% to 90% by weight of polypropylene oxide and from 1% to 20% by weight of silicone, wherein said silicone polyether block copolymer has a molecular weight of from 10000 to 15000.

### DETAILED DESCRIPTION OF THE INVENTION

The compositions of the invention may be aqueous, preferably substantially aqueous. It has been found that when selecting a composition to be used in hair removal devices, it can be particularly desirable to select a composition which is sufficiently thick and viscous that it will not run off the skin or razor after being dispensed. Additionally, moisturizing compositions can be desirable for use in a fluid dispensing hair removal device to allow for multiple benefits, including but not limited to hydration of the hairs prior to shaving, moisturization of skin during the hair removal process, lubrication of skin to reduce friction during the shave, and so forth. Those of skill in the art will understand that moisturization can include hydration of the skin or hair or occlusion of the skin and or hair, or lubrication of the hair or skin to increase glide and reduce friction between the fluid dispensing device and skin.

### Water

The composition of the invention comprises water. In one embodiment, the composition comprises at least about 30% by weight water. In an alternate embodiment, the composition comprises at least about 40% by weight water. In an alternate embodiment, the composition comprises at least about 50%, more preferably at least 60%, even more preferably at least 80% and even more preferably at least 90% by weight water. Compositions having high levels of water enable the device to be used without the necessity for an additional water source to apply or remove the composition from the skin after application.

### Lubricating material: Silicone polyether copolymer

According to the invention, the composition comprises from about 0.1% to about 60%, preferably from about 0.1% to about 20%, more preferably from about 0.1% to 5%, even more preferably from about 0.1% to about 1% by weight of a silicone polyether copolymer or mixtures thereof.

The silicone polyether copolymer comprises from about 1% to 50%, preferably from 1% to 30%, by weight of polyethylene oxide, from about 20% to about 90%, preferably from 20% to 80% by weight of polypropylene oxide and from about 1% to about 20% by weight of silicone. Preferably the silicone polyether copolymer comprises at least about 40%, more preferably at least about 50%, most preferably at least about 60% by weight of polypropylene oxide. In addition, the silicone polyether copolymer preferably comprises at least about 10%, more preferably from at least about 15%, most preferably from about 15% to 30% by weight of polyethylene oxide. Furthermore, the silicone polyether block copolymer comprises from 1% to 20%, preferably 10% to 20%, more preferably about 15% by weight of silicone.

Whilst silicone polyether block copolymers are known in the art to provide a number of benefits such as foaming, defoaming, wetting, deaceration and lubricity, it has been now been surprisingly found that the selection of silicone block copolymers having from 20% to 90% by weight of polypropylene and from 1% to 50% of polyethylene oxide unexpectedly provide improved lubrication whilst ensuring the required level of water dispersion and or solubility verses silicone polyether block copolymers having less or no polypropylene and more polyethylene oxide. Moreover, the use of such silicone block copolymers provides improved adhesion to the skin verses alternative materials such as copolymers of polyethylene oxide and polypropylene oxide. Furthermore, the inclusion of 1% to 20% of silicone by weight of the silicone polyether block copolymer surprisingly provides desirable levels of lubrication despite being present at low levels in the polymer.

The copolymers are block copolymers and may have a pendant graft structure or a linear structure. The silicone polyether block copolymer comprises from 1% to 50%, preferably from 10% to 30%, more preferably about 20% by weight of polyethylene oxide. The silicone polyether block copolymer comprises from 20% to 90%, preferably from 40% to 80%, more preferably from 50 to 80%, most preferably about 65% by weight of polypropylene oxide. The silicone polyether block copolymer comprises from 1% to 20%, preferably 10% to 20%, more preferably about 15% by weight of silicone.

The silicone polyether block copolymer preferably has a ratio of polyethylene oxide units to polypropylene oxide units of from 2.0 to 0.1, more preferably from 0.6 to 0.25. The silicone polyether block copolymer preferably has a ratio of polyethylene oxide units to polypropylene oxide units to silicone units of from 20:65:15.

The silicone polyether copolymer has a molecular weight of from 10000 to 15000. Suitable silicone polyether copolymers are available from Momentive under the Silwets trademark products including L7210.

In one embodiment the lubricating member comprises silicone polyether block copolymer and a water soluble polymer, preferably polyethylene oxide at a weight ratio of from 1:8 to 8:1, preferably from 1:5 to 5:1, more preferably from 1:3to 3:1 and even more preferably from 1:2 to 2:1.

In a preferred embodiment the silicone polyether copolymers suitable for use herein only contain repeating units of silicone, polyethylene oxide and polypropylene oxide. Silicone polyether copolymers comprising additional alkyl chains are preferably excluded.

In one embodiment, preferably the silicone polyether block copolymer is sparingly soluble, preferably soluble or more preferably freely soluble in water according to the United States' Pharmacopeia (USP) definition in 31/NF 26 Vol. 2 General Notices, Page Xvii. According to that definition, sparingly soluble means that 30 to 1000 parts of water are needed to dissolve 1 part solute, soluble means that 10 to 30 parts of water are needed to dissolve 1 part solute and freely soluble means than from 1 to 10 parts of water are needed to dissolve 1 part of solute.

The liquid composition comprising the silicone polyether block copolymer as defined in claim 1 and preferably comprising any optional components may have a coefficient of friction as defined according to the method described herein of 0.0300 or less, preferably of 0.0275 or less, more preferably of 0.0250 or less in order to improve lubrication.

### Thickening Agent

The composition may contain one or more thickening agents, from about 0.1% to about 5%, alternatively from about 0.1% to about 4%, alternatively from about 0.25% to about 3%, by weight of the composition.

Non limiting classes of thickening agents include those selected from the following: Carboxylic Acid Polymers, Crosslinked Polyacrylate Polymers Polyacrylamide Polymers, Polysaccharides, Clays and Gums, and mixtures thereof when appropriate. In one embodiment, compositions of the present invention include a thickening agent selected from carboxylic acid polymers, crosslinked polyacrylate polymers, polyacrylamide polymers, polysaccharides, and mixtures thereof, more preferably selected from carboxylic acid polymers, polyacrylamide polymers, polysaccharides, and mixtures thereof.

Preferred thickening/suspending agents include electrolyte sensitive polymers that are shear thinning when in solution. Shear thinning is property that makes a liquid easy to spread and pump. We have found that electrolyte sensitive polymers have desired performance profiles.

While not wishing to be bound by theory, the electrolyte sensitive polymers interact with the residual surfactant or electrolyte left on the skin and release the lubrication agents and/or suspended conditioning agents for spreading across the razor and across the surface of the skin. Preferred electrolyte sensitive polymers include but are not limited to: Polyacrylamide, Hydroxyethyl Acrylate/Sodium Acryloyldimethyltaurate Copolymer, Sodium Acrylate/Sodium Acryloyldimethyl Taurate Copolymer, Ammonium Polyacrylate, Sodium Acrylate/Acryloyldimethyltaurate/Dimethylacrylamide Crosspolymer, Hydroxyethyl Acrylate/Sodium Acryloyldimethyltaurate Copolymer which can be purchased from Seppic or Carboxylic Acid Polymers (Carbomers) such as Ultrez 10, Carbopol 934, Carbopol 980 and ETD 2050 which can be purchased from Lubrizol or Ammonium Acryloyldimethyltaurate/VP Copolymer, Sodium Acryloyldimethyltaurate/VP Copolymer, Ammonium Acryloyldimethyltaurate/Beheneth-25 Methacrylate Crosspolymer, which can be purchased from Clariant. The most preferred electrolyte sensitive polymer is Polyacrylamide available as Sepigel 305 (Polyacrylamide & C13-14 Isoparaffin & Laureth-7).

### Surfactants

The composition may contain one or more surfactants, from about 0.1% to about 20%, alternatively from about 0.5% to about 15%, alternatively from about 1.0 % to about 12%, by weight of the composition. Non limiting examples of surfactants for use herein are disclosed in McCutcheon's, Detergents and Emulsifiers, North American edition (1986), published by allured Publishing Corporation; and McCutcheon's, Functional Materials, North American Edition (1992). Preferred surfactants are nonionic surfactants/emulsifiers. Non limiting useful surfactants herein include those selected from the group consisting of alkyl glucosides, alkyl polyglucosides, polyhydroxy fatty acid amides, alkoxylated fatty acid esters, sucrose esters, alkoxylated fatty alcohols, amine oxides, and mixtures thereof. Most preferred are alkoxylated fatty alcohols and alkyl glucosides and mixtures thereof.

In one embodiment the composition comprises less than about 5%, or less than about 3%, or less than about 2% of one or more lathering surfactants or soaps. In one embodiment the composition is free or substantially free of lathering surfactants or soaps. A lathering surfactant is defined as a surfactant which when combined with water and mechanically agitated generate a foam or later. Lathering surfactants include anionic and amphoteric lathering surfactants and mixtures thereof.

Anionic lathering surfactants include sarcosinates, sulfates, sulfonate, isethionate, taurates, phosphates, lactylates, glutamates, alkali metal salts of fatty acids (i.e. soaps) having from 8 to 24 carbons, and mixtures thereof.

### Lubricants

The compositions may employ one or more additional lubricants, from about 0.1% to about 8%, alternatively from about 0.1% to about 5%, alternatively from about 0.2% to about 3%, by weight of the composition. Exemplary lubricants include lubricous water soluble polymers, water insoluble particles, and hydrogel-forming (or water swellable) polymers, and mixtures thereof.

Examples of suitable water soluble polymers include polyethylene oxide, polyvinyl pyrrolidone, polyacrylamide, polyhydroxymethacrylate, polyvinyl imidazoline, polyethylene glycol, polyvinyl alcohol, polyhydroxyethymethacrylate, guars, celluloses, modified celluloses and mixtures thereof. In some embodiments, said water soluble polymer is selected from the group consisting of polyethylene oxide, polyethylene glycol, and mixtures thereof.

The preferred water soluble polymers are the polyethylene oxides generally known as POLYOX (available from Union Carbide Corporation) or ALKOX (available from Meisei Chemical Works, Kyoto, Japan). The water soluble polymer, (especially these polyethylene oxides), may have average molecular weights of at least about 20,000, preferably at least about 50,000, more preferably at least about 100,000 or from about 100,000 to about 8 million, preferably from about 300,000 to about 8 million, more preferably from about 1 million to about 5 million, even more preferably about 2 to 3 million. A particularly preferred polyethylene oxide comprises a blend of about 40% to 80% of polyethylene oxide having an average molecular weight of about 5 million (e.g. POLYOX COAGULANT) and about 60% to 20% of polyethylene oxide having an average molecular weight of about 300,000 (e.g. POLYOX WSR-N-750). The polyethylene oxide blend may also advantageously contain up to about 10% (for example about 5%) by weight of a low molecular weight (i.e. MW< 10,000) polyethylene glycol such as PEG-100.

Another suitable lubricant is a copolymer of polyethylene oxide (PEO) and polypropylene oxide (PPO). The PEO/PPO copolymer may have an average molecular weight of at least 5,000, preferably in the range of from 10,000 to 20,000, more preferably from 11,000 to 15,000, even more preferably from 12,000 to 13,000 and even more preferably still from 12,250 to 12,750. The PEO/PPO copolymer may advantageously be a block copolymer, preferably a tri-block copolymer having the sequence: PEO-PPO-PEO, the later commercially available under tradenames such as Pluracare from BASF and Pluronic from Sigma-Aldrich. The PEO/PPO copolymer may have a weight ratio of PEO to PPO (i.e. of ethylene oxide repeat units to propylene oxide repeat units), of from 1000:1 to 1:1000 or from 100:1 to 1:100.

Non limiting useful water insoluble particles may include inorganic particles or organic polymer particles. Hydrogel-forming polymers are typically highly hydrophilic polymers that, in water, form organized three-dimensional domains of approximately nanometer scale. Additional polymer lubricants include: cellulose derivatives such hydroxyalkyl cellulose polymers such as hydroxyethyl cellulose and hydroxypropyl cellulose, carboxymethyl cellulose, and cellulose methyl ether and polysaccharide gums such as, for example, xanthan gum, carrageenan gum, guar gum, locust bean gum, and hydroxypropyl guar gum.

### Sensates

In one embodiment of the invention, the composition may contain sensates, or combinations of sensates. Sensates can be materials that provide the sensation of a thermal change, e.g., heating or cooling. Applicants have found that the addition of sensates using this composition provides longer lasting skin sensation and comfort benefits. Non-limiting examples include: p-Methane-3,8-diol; Isopulegol; Menthoxypropane-1,2,-diol; Curcumin; Menthyl Lactate; Gingerol; Icilin; Menthol; Tea Tree Oil; Methyl Salicylate; Camphor; Peppermint Oil; N-Ethyl-p-menthane-3-carboxamide; N-[4-(Cyanomethyl)phenyl]-2-isopropyl-5-methylcyclohexane-carboxamide; Ethyl 3-(p-menthane-3-carboxamido)acetate; 2-Isopropyl-N,2,3-trimethylbutyramide; Menthone glycerol ketal, and mixtures thereof.

### Optional Ingredients

The composition may further comprise additional optional ingredients. Suitable additional optional ingredients include perfume, preservatives, chelants, sensates (e.g. menthol), desquamation actives, anti-acne actives, anti-wrinkle/anti-atrophy actives, anti-oxidants/radical scavengers, flavonoids, anti-inflammatory agents, anti-cellulite agents, topical anesthetics, tanning actives, skin lightening agents, skin soothing and healing actives, antimicrobial actives, sunscreen actives, visual skin enhancers, humectants and moisturizing agents (e.g., glycerin, glycols, sorbitol), skin feel actives such as cationic polymers and the like. Such optional ingredients are described more fully in U.S. Application Serial No. 11/367,918, filed March 3, 2006. Preferred additional optional ingredients include salicylic acid, opacifiers (e.g. mica and titanium dioxide), perfume, hydrophilic conditioning agents (e.g., glycerin) and skin sensates (e.g. menthol).

Opacifiers may be added to the shave care composition of the present invention. Opacifiers may be either inorganic or organic compounds. Inorganic opacifiers include, for example, titanium dioxide, zinc oxide, talc, mica or coated mica (with oxides of titanium, tin, or iron or bismuth oxychloride), magnesium aluminum silicate, bismuth oxychloride, or other minerals. These compounds can be added as powders, dispersions, or complexes. Organic opacifiers include, for example, opaque emulsions (e.g., containing Styrene/PVP copolymer, vinyl polymers, or latexes), metal salts of amines containing 14-20 carbon atoms per molecule, alkanolamides containing 14-20 carbon atoms per molecule, organic alcohols containing 14-20 carbon atoms per molecule, insoluble salts of stearic acid, glycol mono-or distearates, propylene glycol and glycerol monostearates and palmitates. Combinations of these opacifiers can also be used. The opacifying additive is typically included in an amount of about 1 to about 6%, preferably about 2 to about 5%, by weight of the composition.

For shaving personal care composition embodiments typically applied to the skin before shaving, the compositions may comprise from 40% to 95%, preferably 60% to 95% by weight of water and optionally from 1% to 6% by weight of a volatile post foaming agent. Suitable post foaming agents are selected from aliphatic hydrocarbons having 4 to 6 carbon atoms such as n-pentane, isopentance, neopentane, n-butane, isobutene and mixtures thereof. The personal care compositions may be formulated as aerosol foams, a post foaming gel, a non-aerosol gel or lather and may be packaged in suitable dispenser for dispensing such compositions.

The liquid compositions for use with a hair removal device and or before or after shaving may comprise less than 5%, preferably less than 1% by weight and more preferably is/are substantially free of soap (i.e. salts of fatty C4 to C30 acids) or lathering surfactant as defined hereinabove.

### Composition Dispensing Device

The composition dispensing device of the present invention can be any such device which allows the present composition to be dispensed therefrom during the hair removal process. Examples of many types of composition dispensing devices are known. In one embodiment the composition dispensing device is s device having a plurality of shaver heads with rotary blade cutters. In another embodiment, the composition dispensing device is a composition dispensing hair removal device preferably a razor comprising one or more safety razors.

In one embodiment, the device is an automatic vibrating and/or dispensing razors. For example, U.S. 2008-0289185 discloses a razor comprising a fluid delivery system having an electrically actuable dispensing device to control delivery of the fluid, and a control device for controlling actuation of the dispensing device. The control device is in proximity or is touch sensitive and includes a sensor element arranged to be brought into contact with or into close proximity to the skin being shaved during the performance of a shaving stroke. Another suitable device is disclosed in U.S. 7441336, which discloses an automated razor which has a control device allowing for automated vibration or dispensing when a certain environmental condition is met, such as proximity or touch with the intended surface or electrical conductance. These types of automated dispensing devices can be particularly useful so that the composition can be dispensed at the desired time onto the skin, thereby minimizing wasted product which could otherwise be captured within the device head or elements thereof. Further, using an automated device may be advantageous as users may have a difficult time deciding when to trigger the dispensing action during the hair removal process. For example, they may accidently trigger the dispensing too early or excessively, causing an undesirably large amount of composition to come out and potentially miss the intended surface for treatment. Automated dispensing, however, is not required, as manual dispensing systems can also be useful for certain purposes.

Non-limiting examples of other composition dispensing devices suitable for use with the present invention include those disclosed in U.S. 2006/00240380 U.S. 2007/0084074; U.S. 7,127,817; U.S. 7,121,754; and U.S. 6,789,321. In some of these examples, the product can be dispensed at or about the vicinity of the device head (commonly a razor cartridge).

In one particularly useful embodiment, the composition can be dispensed through an elongated elastomeric contact region comprising a dispensing orifice which allows the composition to spread in a wide strip onto the surface, such as the lumens described in U.S. 2006/00240380 (see *inter alia* Fig. 17). Similar dispensing systems have also been described in U.S. Serial No. 61/340299 to Royle et al, filed March 15, 2010. The lumens or dispensing orifice can be particularly useful when dispensing the present composition given the specific rheology (thickness and viscosity) desired for use herein. In particular, since the composition is desirably thick and viscous, an elongated dispensing orifice or a plurality of orifices oriented to dispense a wide yet thin layer of the composition may be desired. Advantageously, the layer of composition deposited does not excessively run or drip off the surface prior to the treated surface coming in contact with the razor blade or other hair removal head. Further, where the composition is clear or translucent, the user can easily see where they are shaving so they can have fine control to make clean shaven areas such as a beard line. This can be particularly beneficial over devices comprising shaving heads with rotary blades which would not be as capable of allowing for fine control to make clean shaving lines.

In one embodiment, the composition dispensing device comprises a handle connected to a hair removal head, the handle further comprising a cavity for housing said composition disposed within the handle, and an actuator adapted to displace the composition from the cavity to a fluid dispensing member, and wherein said fluid dispensing member comprises an elongated elastomeric contact region forming at least one dispensing orifice which is generally perpendicular to a transverse centerline of the handle. The fluid dispensing member may extend to or adjacent to the bottom portion of the hair removal head allowing for direct contact or near direct contact to a user's skin during application of the hair removal head to skin, such as during a shaving stroke. The fluid dispensing member comprises a fluid dispensing member comprising at least one elongated elastomeric contact region. In one embodiment, the fluid dispensing member also comprises a one-way valve, which can be formed from said elongated elastomeric contact region. Those of skill in the art will understand that the elastomeric material forming the flap valve, slit valve or duckbill valve is such that upon contact with skin, the valve will deform and allow said one or more dispensing orifice(s) to allow fluid to dispense.

In one embodiment, said elongated elastomeric contact region forms a one-way valve which will only allow the composition to exit so entry of undesirable contaminants into the plumbing or cavity of the device is minimized. Non-limiting examples of suitable one-way valves include: check valves such as diaphragm check valves, swing check valves or tilting disc check valves, stop-check valves, lift-check valves, flap valves, slit valves, and/or a duckbill valve. In one embodiment, the fluid dispensing member forms at least one, but optionally two or more dispensing orifices at the dispensing end of the elongated elastomeric contact member for delivering said fluid from the cavity onto skin prior to hair removal. To prevent the fluid from leaking, the fluid flow path, along with any or all of the dispensing orifice(s) may comprise a check valve.

In another embodiment, the cavity can have multiple compartments. For example the cavity can have a first compartment containing said composition, and wherein said cavity forms a second compartment for a second composition. In another embodiment, the device comprises multiple cavities, where different compositions can be contained therein. In one embodiment, the second composition is selected from the group consisting of an aftershave, a lotion, a balm, a fragrance, or a mixture thereof. Examples of known dispensing devices which allow for multiple compositions to be contained therein include: U.S. 6,986,207; U.S. 5,855,066; and U.S. 4,129,942. In one embodiment, the composition can be used with a device capable of dispensing multiple compositions such as therein described.

### Hair Removal Head

The hair removal head can include a wide scraping surface such as where the composition dispensing device is used with a depilatory, or a razor cartridge where the device is a shaving razor. The hair removal head may be replaceable or pivotally connected to a cartridge connecting structure. In an aspect, the cartridge connecting structure includes at least one arm to releasably engage the hair removal head.

Where the hair removal head is a razor cartridge the cartridge may also include multiple blades. For example, U.S. 7,168,173 generally describes a Fusion® razor that is commercially available from The Gillette Company which includes a razor cartridge with multiple blades. Additionally, the razor cartridge may include a guard as well as a shaving aid. A variety of razor cartridges can be used in accordance with the present invention. Nonlimiting examples of suitable razor cartridges, with and without fins, guards, and/or shave aids, include those marketed by The Gillette Company under the Fusion®, Venus® product lines as well as those disclosed in U.S. 7,197,825, U.S.6,449,849, U.S.6,442,839, U.S.6,301,785, U.S.6,298,558; U.S.6,161,288, and U.S. 2008/060201.

### Fluid Dispensing Member

The fluid dispensing member may comprise an elongated elastomeric contact region. Non-limiting examples of suitable elongated elastomeric contact regions include: dual slit or duckbill valves such as those described in U.S. 2006/00240380 in FIGs 1 - 9 and paragraphs 52 to 58. The present invention, however, does not require dual lumens to be present. Further, the present fluid dispensing member is designed to deliver fluid away from, preferably preceding, the head area of the devices disclosed in the art. By delivering fluid prior to the skin contacting the hair removal head, it allows for broader spreading of the fluid and additional time where the fluid can come into contact with the folds and crevices within the skin. In one embodiment, the fluid dispensing member further comprises a non-elastomeric portion which can precede said elongate elastomeric contact member. The non-elastomeric portion can be formed of the same material as used to form any part of the remainder of the handle. In one embodiment, the one-way valve is not formed in said elongated elastomeric contact member. The one-way valve can be formed in the non-elastomeric portion of said fluid dispensing member or in any portion of said fluid flow path, such as in the supply channel, at the opening, and/or in the fluid dispensing path.

"Elongated" as defined herein means, that the object has a major and a minor axis, wherein the major axis is at least 10 times larger than the minor axis. The elongated portion of the fluid dispensing member has a width (major axis) which is at least 10 times larger than the height. In one embodiment, the width measures from about 2 cm to about 15 cm, alternatively from about 3 cm to about 10 cm, alternatively from about 4 cm to about 8 cm. In another embodiment, the height of the elongated one-way elastomeric valve is about 1 cm, alternatively about 0.5 cm, alternatively from about 0.2 cm, alternatively the elongated one-way elastomeric valve is biased to be in a sealed orientation when not in use. Those of ordinary skill in the art will understand that a check valve may be used in embodiments where the elongated one-way elastomeric valve is not sealed when not in use to minimize product leakage. In another embodiment, the fluid is chosen such that even if the height of the valve is such that it remains unsealed and open when not in use, the fluid is sufficiently viscous and thick that it will not undesirably leak when not being actuated by the user.

"Elastomeric" as defined herein means a material which is generally flexible and deformable. In one embodiment, the elongated elastomeric contact member has a young's modulus of elasticity of from about 0.01 GPa to about 3.5 GPa, alternatively from about 0.02 GPa to about 2 GPa, alternatively from about 0.05 GPa to about 1 GPa, alternatively from about 0.1 GPa to about 0.5 GPa. Non-limiting examples of suitable materials which can be used to form the elastomeric contact member include rubber, silicone, Teflon, and polyethylene. Without intending to be bound by theory, it is believed that by providing an elastomeric material in the fluid dispensing member at the point where the fluid dispensing member would contact skin is particularly useful as it decreases irritation onto skin from a non-elastomeric fluid dispensing member. Further, the elastomeric material allows the tip of the fluid dispensing member to deform to better engage the non symmetric shape of body parts. In one embodiment, the elastomeric material used has a shore hardness of from about 30 to about 40 D units.

"Slit valve" as defined herein means that the valve comprises a closed slit and flow is provided by flexing or deformation of the elastomeric material which causes the slit to open. In general the slit valve is a single piece construction which is free of moving parts. "Duckbilled valve" as defined herein is a type of slit valve, wherein one end of the valve is stretched over the outlet of the fluid dispensing path, conforming itself to the shape of the path, usually round. The other end, the duckbill, retains a natural flattened shape. When a fluid is pumped through the fluid dispensing path, the duckbill's flattened end opens to permit the pressurized fluid to pass. When pressure is removed, however, the duckbill end returns to its flattened shape, preventing backflow. Other check valves referred to herein are known in the art.

"Generally perpendicular" as defined herein means that the lateral dimension of the elongated elastomeric contact region forms an angle which is from about 75° to about 90° as measured against the transverse centerline passing through the handle. Since the elongated elastomeric contact region is generally elastic and therefore deformable in nature, this angle is measured when the fluid dispensing region is at rest and not deformed or otherwise manipulated by a user.

The elongated elastomeric contact region comprises a contact point where the fluid dispensing member engages the surface (skin). In one embodiment, the contact point forms a straight line. In another embodiment, the contact point forms a concave or convex line. Similarly, "generally parallel" as defined herein means that the two straight lines formed through said objects are parallel or form an angle of from about 0° to about 15° when in a resting position.

The fluid flow path terminates at at least one fluid dispensing orifice. In one embodiment, more than one fluid dispensing orifice is provided. The fluid dispensing orifice is formed of the elastomeric material used to form the elongated elastomeric contact member. Preferably, the fluid dispensing orifice has a wide and narrow shape similar to the fluid dispensing member but the fluid dispensing orifice can also be of different shapes. In one embodiment, the fluid dispensing member comprises a plurality of fluid dispensing orifices which are spaced out along the width of the elongated elastomeric contact member such that when fluid is dispensed, a wide flat application of fluid can still be deposited. The fluid dispensing orifices can be round, oval, triangular, square, rectangular in shape, or combinations thereof. In one embodiment, the portion of the elongated elastomeric contact member leading up to the fluid dispensing orifice forms a tapered exit channel, the tapering can be tapered in, or tapered out. Without intending to be bound by theory, it is believed that a tapered exit channel, particularly one that tapers out so the cross sectional area of the orifice is larger than the cross sectional area of the channel leading to the orifice, can be useful to ease in removal of any fluid which can reside in the vicinity of the orifice after use. A tapered in exit channel may be useful to minimize exposure of the fluid to contact with air, thereby minimizing fluid dry out.

### Actuator

As explained above, the actuator can be manual or automatic pump (battery powered or via an external power source). The pump includes a wall, either movable or rigid, upon which force is acted upon to move the fluid through. In the case of a movable wall, the movable wall may be located on one or more of an upper or lower surface of the handle. For a rigid wall, the force causes the movement of non-rigid sidewalls of the pump to move a fluid through to the channel.

In one embodiment, the actuator is a manually-actuated pump which can reside on the handle. In another embodiment, the actuator is automated and can be powered by a battery or external power source. In yet further embodiments, the actuator comprises a pump which is actuated by movement of the shaving head (such as where depression of the head or rotation of the head about the pivot axis), actuates the pump. In yet another embodiment, the fluid dispensing member itself can be spring loaded and retractable upon contact with a surface such as skin such that the movement of the fluid dispensing member can act as the actuation to actuate the pump. Those of skill in the art will understand that in this type of embodiment, it could be preferred to have the elongated elastomeric contact region extend beyond the general plane of the shaving head such that when the device is brought into contact with a surface (such as skin) the fluid dispensing member will be pushed back towards the razor prior to surface contact with the shaving head. The movement of the fluid dispensing member can then actuate the pump permitting fluid to escape or be driven out of the cavity through the fluid flow path, out of the at least one dispensing orifice, ultimately onto the skin.

In one embodiment the composition dispensing device includes a handle and a hair removal head, such as a disposable razor cartridge. The composition dispensing device can be a wet or dry, manual or powered razor, having straight or rotary blades. In addition, the composition dispensing device can be used with a depilatory, therefore not requiring the use of razor. The handle has a length that extends from a proximal end to a distal end and a transverse centerline which runs along the central axis of the handle. The handle comprises a cavity for housing a fluid disposed within the handle, and an actuator adapted to displace the fluid from the cavity preferably through a supply channel to an opening formed in said handle, such as towards the proximal end of the handle.

The composition dispensing device may include a fluid dispensing member comprising an elongated elastomeric contact region forming at least one dispensing orifice in fluid communication with said opening formed in said handle. Said elongated elastomeric contact region comprises a lateral dimension, which is generally perpendicular to said transverse centerline of the handle. The hair removal head also has a lateral dimension which his generally perpendicular to said transverse centerline. In one embodiment, the ratio of the lateral dimension of the elongated elastomeric contact region to the lateral dimension of the hair removal head is from 1:10 to about 1.5:1, alternatively from about 0.5:1 to about 1:1. Without intending to be bound by theory, it is believed that by providing an elongated elastomeric contact region which is laterally sized with respect to the hair removal head as recited herein, the fluid dispensed from said at fluid dispensing member covers a sufficiently broad portion of said hair removal head to provide suitable product spreading over skin and into cracks and corners of the skin. The fluid dispensing member is in fluid communication with said cavity via said opening, forming a fluid dispensing path, wherein said supply channel and said fluid dispensing path form a fluid flow path.

In one embodiment, the device includes at least one, one-way valve located at some point along said fluid flow path. As explained above, in some embodiments, said elongated elastomeric contact point forms said one-way valve. Additional one-way valves can also be included along the fluid flow path as desired.

The actuators are typically manual pumps but automatic pumps can also be included. The actuators, which can be manual or automatic, and may include pumps which can be stacked (and substantially flat) components and particularly a movable wall that acts to activate the flow of fluid from the cavity through channel and to the opening. A pump suitable for use in the present invention is disclosed in U.S. 5,993,180. In particular, this pump includes a pump chamber bounded by the movable wall, an inlet channel and an outlet channel, both of which are connected to the pump chamber, an inlet valve for closing the inlet channel, and an outlet valve for closing the outlet channel. In most instances, the pump may be actuated by the pressure exerted by a user's finger such that the user may easily determine the requisite amount of fluid for one or more shaving strokes. Because the valves of the pump are automatically opened when pressure is applied by the user's finger pressure, the fluid can be dispensed in controlled and metered quantities without relying on judgment or dexterity of the user. It is also possible to place one or more movable walls of the pump on an upper surface or lower surface of the razor depending on a user's preference. The actuator provides a feed into the cavity. This feed can be application of pressure or another impulse which will drive fluid through said fluid flow path out to the fluid dispensing member. The actuator may alternatively has a receiving chamber where fluid is transferred prior to entering the supply channel and passing into the fluid dispensing member. These and other actuators and pumps which are known in the art for use in personal care devices which dispense fluids can be used in accordance with the present invention.

The cavity or at least a container/sachet within the cavity contains the fluid to be dispensed during the hair removal process. In one embodiment, the fluid in the cavity or container is refillable or replaceable.

In another embodiment fluid dispensing member is pivotably attached to said handle via a hinge member positioned on said handle. In one embodiment, a portion of the fluid flow path, such as the fluid dispensing path can be exposed upon exiting said opening formed in said handle.

In another embodiment of the composition dispensing device, the fluid dispensing member may deform. The fluid dispensing member may extend beyond the general facial plane formed by said hair removal head. Since the fluid dispensing member comprises an elastomeric contact region, the portion of the fluid dispensing member which extends beyond the plane of the hair removal head would deform, when the device comes in contact with a surface, such as skin. A fluid dispensing member is deformed when the device is in an "in-use" position, allowing fluid to exit the at least one dispensing orifice formed in the elongated elastomeric contact region. The elongated elastomeric contact region can flex toward the hair removal head, flex away from the hair removal head and can even come into contact with a portion of the hair removal head, all depending on the movement of the device with respect to the surface. A volume of fluid is deposited onto the skin and the hair removal head is moved in a downward trajectory along the skin to remove hairs which have been treated with said fluid. Further, the one-way valve shown is positioned along the fluid flow path but not at the point where the fluid dispensing member forms said at least one dispensing orifice.

In one embodiment, the cartridge attaches to the rear surface of a housing by a cartridge connecting structure. The cartridge connecting structure may include one or more arms that extend to provide pivotal support of the housing. Alternatively, the cartridge connecting structure may include an ejection mechanism (e.g., a button) to disengage the housing from the cartridge connecting structure. Non-limiting examples of suitable housings and cartridge connecting structures are described in: U.S. Patents 7,197,825, 5,822,869, 6,161,287, and 5,784,790.

The razor cartridge may also include a guard and or lubricating strip. The guard is useful for stretching the skin's surface immediately prior to engagement with the blade or a first blade (when more than one blade is present). This guard may typically comprise an elastomeric member to allow for an engagement that is comfortable to a user. Typically, the elastomeric material used is a block copolymer (or other suitable materials), preferably having a durometer between 28 and 60 Shore A.

The lubricating strip, on the other hand, provides an additional treatment to the skin after contact between the fluid and the skin has occurred. The lubricating strip may contain the same or additional skin ingredients to those that are present in the fluid. The cartridge connecting structure may be releasably engaged from the handle, as disclosed for example in U.S. Patents D533,684, 5,918,369, and 7,168,173. This disengagement of these two components allows for replacement of razor cartridges as the continued use of such cartridges causes blade dulling. Thus, such cartridges are replaceable and disposable at will by the user.

In another embodiment the hair removal head is a razor cartridge with a plurality of blades and a lubricating strip shaving aid as well as a guard. The razor cartridge may have a lateral dimension which can measure any length typically used for conventional straight blade wet razor cartridges, for example from about 2cm to about 10cm, alternatively from about 3cm to about 8cm, alternatively from about 4cm to about 7cm. Said elongated elastomeric contact region comprises a lateral dimension which is generally perpendicular to said transverse centerline of the handle. In this embodiment, the device comprises two fluid dispensing orifices. Those of skill in the art will understand that different fluid dispensing orifice configurations are within the scope of the invention. The two fluid dispensing orifices may be equal in length and are positioned linear to one another. The lengths can vary and the orifices can be staggered so they do not sit on the same line. Further, although the at least one fluid dispensing orifice is generally parallel to the angle of the razor cartridge and/or blades, the orifice can be angled. The lateral dimension of the at least one fluid dispensing orifice is measured as the greatest lateral distance covered by the orifice, regardless of the angle upon which the orifice sits with respect to the razor cartridge and/or blades. In another embodiment, the at least one fluid dispensing orifice can have a curved or wavy line shape. In one embodiment, the ratio of the lateral dimension of the at least one fluid dispensing orifice to the lateral dimension of the hair removal head is from about 1:10 to about 1:1, alternatively from about 1:5 to about 1:2.

In one embodiment the elongated elastomeric contact member is with a transverse central axis. The elongated elastomeric contact member, being deformable and elastic in nature can twist, bend, compress and stretch as needed. In this embodiment, the elongated elastomeric contact member has a rotation path showing the ability of the elongated elastomeric contact member to rotate about said transverse central axis. In this embodiment, the portion of the elongated elastomeric contact member which forms the at least one fluid dispensing orifice is in a sealed position, and has a greater lateral dimension than the portion of the elongated elastomeric contact member which would be closer to the handle. Those of skill in the art will understand that the elongated elastomeric contact member can have a constant, increasing or decreasing lateral dimension as the lateral dimension is measured from the distal end to the proximal end (towards the handle).

In another embodiment, both the tip of the fluid dispensing member and the at least one fluid dispensing orifice are concave shaped so they can contour to body parts easier. The at least one fluid dispensing orifice has a lateral dimension. This could be particularly preferable for female composition dispensing devices which are designed for use on the leg or arms. In this embodiment, the hair removal head has a scraping edge. The hair removal head can also be a razor cartridge as described above.

In one embodiment, the hair removal head has a skin contacting edge which is flat, concave or convex. Those of skill in the art will understand that different shapes for the skin contacting edge can be preferred based on the desired part of the body upon which the device is intended for use. For example, a composition dispensing device intended for use on the face may have an applicator having a straight edge. A composition dispensing device intended for use on legs may have an applicator having a concave edge. Non-limiting examples of suitable head configurations are disclosed in U.S. Patent Nos. D399,601, D203,892, and 651,420 ; U.S. 3,088,470, U.S. 3,858,985, and U.S. 2004 0168743A1; WO 97/18043A1 and GB1390153..

In another embodiment the fluid dispensing member has an angled and tapered distal region (extending away from the handle). A fluid dispensing orifice is in fluid communication with the fluid flow path. In one embodiment, a check valve is provided along the fluid flow path. In another embodiment, the fluid dispensing orifice can include a flap or be designed to close when not in use. The fluid dispensing orifice could then act as a one-way valve as described above. In one embodiment the fluid flow path has a constant cross sectional area or a varying cross sectional area. The fluid flow path is tapered as it approaches the fluid dispensing orifice.

In one embodiment, the fluid dispensing orifice has a width of from about 2cm to about 15 cm, alternatively from about 3cm to about 10cm, alternatively from about 4 cm to about 8 cm. Where numerous fluid dispensing members are provided, the width can be even smaller, as low as about 0.2cm, or about 0.5cm, or about 1cm. The width of the fluid dispensing orifice is preferably 0cm when the device is in a sealed state (not in use) but the width can change when the orifice is opened and can be from about 0.02cm to about 0.5cm, alternatively from about 0.05cm to about 0.3cm, alternatively from about 0.1cm to about 0.2cm. In one embodiment, the fluid dispensing orifice is not 0 cm when not in use. In this embodiment, a check valve can be included somewhere along the fluid flow path to control movement of the fluid before it reaches the fluid dispensing orifice. In another embodiment, the fluid dispensing orifice comprising a width of from about 0.5mm to about 10mm, or from about 1mm to about 3mm, and a length of from about 20mm to about 80mm, or from about 30mm to about 70mm, alternatively from about 40mm to about 50mm.

### Methods of Use

As explained above, the present device is designed for use in the hair removal process, such as when shaving. One embodiment of the present invention provides for a method of removing hair from skin comprising the steps of: providing a composition dispensing device containing the moisturizing personal care composition described herein; actuating said composition dispensing device to dispense said composition; contacting said composition onto a portion of skin to be treated to form a prepared surface; and contacting said prepared surface with the composition dispensing device to form a treated surface.

Another embodiment further comprises a step of wetting said portion of skin to be treated either before contacting said composition onto a portion of skin or after contacting said prepared surface with the composition dispensing device to form a treated surface. The process can also include a step of leaving the treated surface as is, without further washing or rinsing, after the hair removal step.

In another embodiment, the composition is dispenses from the device directly onto skin from the dispensing member of said composition dispensing device. This step can be by manually triggering an actuator, or by an automated control device which senses when the device is in proximity or in contact with the surface to be treated. The composition could also be dispensed onto a portion of the device which is then contacted to the skin to apply the composition but this is not necessary where dispensing directly on to skin is possible.

In yet another embodiment, the steps of contacting said composition onto the skin and contacting said treated surface with the razor blade can occur simultaneously.

In one embodiment, the device is used in a dry shave context where water or other shave preparations are not used to pre-wet the skin. Water can still be used, however, after the dry shave to wash of any shave debris and remaining moisturizing composition. Yet another embodiment provides for a further step of applying a second skin care composition onto the treated surface, such as a post-shave composition. These and other methods of use of the present device in a grooming context are within the scope of the present invention.

### Exemplified compositions:

### I. Examples

The following examples were made according to the tables below by dissolving the listed ingredients into water using gentle agitation with an overhead stirrer until a solution was formed.

| **Ingredient** | **Inventive Example (w/w%)** | **Comparative Example 1 (w/w%)** | **Comparative Example 2 (w/w%)** | **Comparative Example 3 (w/w%)** |
|---|---|---|---|---|
| Silwet L7210 | 1.0 | - | - | - |
| Silwet L7200 | - | 1.0 | - | - |
| Silwet L7220 | - | - | 1.0 | - |
| Silwet L7230 | - | - | - | 1.0 |
| Polyox N-750 * | 0.5 | 0.5 | 0.5 | 0.5 |
| Water | 98.5 | 98.5 | 98.5 | 98.5 |
| Total | 100.00 | 100.00 | 100.00 | 100.00 |

| **Ingredient** | **Comparative Example 4 (w/w%)** | **Comparative Example 5 (w/w%)** | **Comparative Example 6 (w/w%)** |
|---|---|---|---|
| Silwet L7280 | 1.0 | - | - |
| Silwet L7600 | - | 1.0 | - |
| Silwet L7602 | - | - | 1.0 |
| Polyox N-750 * | 0.5 | 0.5 | 0.5 |
| Water | 98.5 | 98.5 | 98.5 |
| Total | 100.00 | 100.00 | 100.00 |

| | | | |
|---|---|---|---|
| *Average molecular weight of 300,000, supplied by Dow Corning; All silwet materials supplied by Momentive | | | |

Silicone polyether block copolymer composition from Momentive supplier information

| **Ingredient** | **Silicone % w/w*** | **PPO % w/w*** | **PEO %w/w*** |
|---|---|---|---|
| Silwet L7210 | 15 | 65 | 20 |
| Silwet L7200 | 34 | 22 | 44 |
| Silwet L7220 | 25 | 55 | 20 |
| Silwet L7230 | 24 | 44 | 30 |
| Silwet L7280 | 38 | 33 | 29 |
| Silwet L7600 | 25 | 0 | 75 |
| Silwet L7602 | 60 | 0 | 40 |

The coefficient of friction (COF) of each of the above solutions was measured using an industry standard rotational tribometer, MTM2 machine from PCS Instruments. The device has a specimen disc mounted on a vertical shaft within a stainless steel test fluid reservoir. A ball is end mounted onto a pivoting shaft and is automatically loaded against the specimen disc at the start of the test. The test fluid is filled into the reservoir. DC servomotors rotate the disk with accurate speed and the traction force applied to the ball specimen is measured with a high sensitivity force transducer, with additional sensors to measure applied load, lubricant temperature and the electrical contact resistance. From this data the coefficient of friction is calculated.

For the above fluids the following conditions were used;
Ball: ¾ inch stainless steel ball (supplied by PCS Instruments)
Specimen disc: 46mm diameter PDMS elastomer disc (material supplied by PCS Instruments). Test Load: 5N; Test Speed: 150 mm/s (shave relevant speed); Temperature: 25°; and Specimen fluid: 40 ml.

### Results:

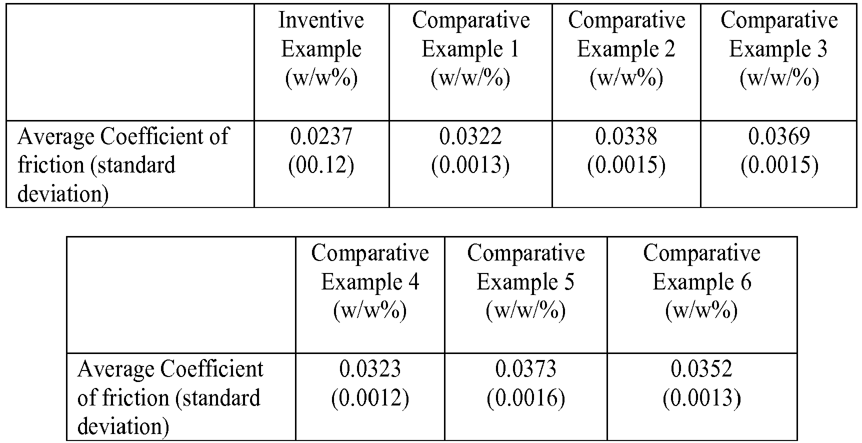

The data clearly shows that the composition containing the silicone polyether copolymer having a low level of silicone and polyethylene and a high level of polypropylene oxide in combination with a water soluble polymer (Inventive example 1) as claimed significantly reduces the coefficient of friction verses polymers with higher levels of silicone and or low levels of polypropylene oxide an higher levels of polyethylene oxide in combination with a water soluble polymer (comparative examples 1-6).

### II. Examples

The following examples were made according to the table below by dissolving the listed ingredients into water using gentle agitation with an overhead stirrer until a solution was formed. A 15 legged experiment was designed to evaluate the above three variables. Silwet 7210 was used in all the examples. The remainder of the formula was DI water. Each of the 15 legs was tested on the MTM2 tribometer using a disc speed range 500 - 1 mm s⁻¹ and a new PDMS disc for each sample. Each solution was repeated four times and a mean COF (and standard deviation) was calculated from the speed region of 90-200mms.

| | **Polyox N750 0.3MM % W/W** | **Polyox N12k 1.0MM %w/w** | **Polyox N60K 2.0MM % W/W** | **Polyox 301 4.0MM % W/W** | **Silwet % W/W** | **Av. COF (sd)** |
|---|---|---|---|---|---|---|
| 1 | 0.2 | - | - | - | 0.07 | 0.0458 (0.0029) |
| 2 | 1.0 | - | - | - | 0.2 | 0.0416 (0.0025) |
| 3 | 1.0 | - | - | - | 0.5 | 0.0396 (0.0018) |
| 4 | - | 0.2 | - | - | 0.04 | 0.0409 (0.0029) |
| 5 | - | 0.6 | - | - | 0.3 | 0.0335 (0.0012) |
| 6 | - | 1.0 | - | - | 0.2 | 0.0325 (0.0011) |
| 7 | - | - | 0.2 | - | 0.04 | 0.0359 (0.0011) |
| 8 | - | - | 0.2 | - | 03 | 0.0322 (0.0009) |
| 9 | - | - | 0.6 | - | 0.2 | 0.0302 (0.0009) |
| 10 | - | - | 1.0 | - | 0.2 | 0.0315 (0.0013) |
| 11 | - | - | 1.0 | - | 0.5 | 0.0287 (0.0010) |
| 12 | - | - | - | 0.2 | 0.07 | 0.0335 (0.0012) |
| 13 | - | - | - | 0.6 | 0.12 | 0.0311 (0.0011) |
| 14 | - | - | - | 0.6 | 0.3 | 0.0343 (0.0016) |
| 15 | - | - | - | 1.0 | 0.33 | 0.0301 (0.0015) |

The above results were then modeled in order identify a preferred formula to reduce the coefficient of friction. Sensory testing was then performed on this preferred formula versus the corresponding non-silicone polyether copolymer containing control to demonstrate the performance improvement.

Sensory testing was conducted upon a naive panel (N=5) with 3-overlapping strokes being performed on their forearms. The order in which each panelist received the products and the first forearms used were randomized. The procedure used was as follows;
1. Wash both forearms thoroughly with warm water and soap to remove any oils or moisturizers that may already be on the skin.
2. Mark out a 15cm x 5cm area on the inner forearm using the template provided and evenly apply 0.1mL amount of fluid.
3. After 3 overlapping strokes using plastic plaque of size (12mm x 40mm) and immediately rate:
   a. Not (0) - Very Glidey (10)
   b. Draggy (0) - Very lubricated (10)
4. Using index finger on forearm rate:
   c. Not Smooth (0) - Extremely Smooth (10)
5. Rinse fore-arm, pat dry and wait for 2mins. Then re-rate smooth attribute as above.

| Property | Glide | Lubricated | Smooth (during & after use) |
|---|---|---|---|
| Test Formula: 0.64% w/w 2MM Polyox N60K + 0.24% w/w Silwet L7210 Control :0.64% w/w 2MM Polyox N60K | 0.8 | 0.6 | 0.4 and 0.6 |

The sensory data indicates a consumer relevant measurable improvement on improved glide, less drag and a smooth feel during and after use verses a control comprising only 0.64% polyethylene oxide (2MM Polyox).

| **III Composition examples Ingredient** | **Example 1 %w/w** | **Example 2 % w/w** | **Example 3 % w/w** |
|---|---|---|---|
| Deionised water | Qs | Qs | Qs |
| Carbopol ETD 2020¹ | 0.500 | - | |
| Ultrez 21 ¹ | - | - | 0.40 |
| Sepigel 305 ² | | 2.0 | |
| DMDM Hydantoin and butyl carbamate | 0.400 | 0.400 | 0.3 |
| Glycerine | 1.000 | 1.000 | 1.0 |
| Panthenol | 0.500 | 0.500 | 1.0 |
| Disodium EDTA | 0.250 | 0.250 | |
| Perfume | 0.150 | 0.150 | 0.0 |
| Silwet L7210 ³ | 0.5 | 0.24 | 0.8 |
| PEG 45M ⁴ | - | 0.64 | 0 |
| Triethanolamine | 0.680 | 0.680 | - |
| Sodium hydroxide | - | - | 0.17 |
| Iodopropynyl butylcarbonate | - | - | 0.09 |
| Phenoxyethanol | - | - | 0.5 |

| | | | |
|---|---|---|---|
| Supplied by: ¹ Lubrizol, ² Seppic, ³ Momentive, ⁴ Dow Chemicals | | | |

This formulation is made as follows: Heat the water and glycerine while stirring (at about 200rpm) to 55°C. Then add the disodium EDTA and PEG45M if present and and continue stirring at 55°C until it is fully dissolved. Then add and carefully disperse the thickener (Carbopol, Ultrez, Sepigel) while stirring (at about 250rpm). Remove from the heat and add the triethanolamine or sodium hydroxide if required and continue to stir at 200rpm. Then add the panthenol while continuing to stir at 200rpm. When the temperature reaches 45°C, add the preservatives and continue to stir for 5 minutes. Lastly, add the Silwet L7210 and perfume and continue stirring for about 5 minutes, followed by 1 minute of high shear (at about 7500rpm).

| **Ingredient** | **Example 4 (%)** | **Example 5 (%)** |
|---|---|---|
| Sorbitol 70% Solution | 0.97 | 0.00 |
| Glycerin | 0.49 | 0.00 |
| Hydroxyethyl cellulose | 0.49 | 0.00 |
| PEG-90M | 0.06 | 0.00 |
| PEG-23M | 0.05 | 0.00 |
| Palmitic acid | 7.53 | 0.00 |
| Stearic Acid | 2.53 | 6.27 |
| Glyceryl Oleate | 1.94 | 0.00 |
| Triethanolamine (99%) | 5.88 | 3.23 |
| Sodium Lauryl Sulfate | 0.00 | 0.78 |
| Laureth-23 | 0.00 | 2.07 |
| Silwet L7210 | 0.50 | 0.05 |
| Fragrance | 0.78 | 0.50 |
| Dye | 0.01 | 0.00 |
| BHT | 0.05 | 0.02 |
| Isopentane (and) Isobutane | 2.85 | 0.00 |
| Butane (and) Propane | 0.00 | 3.00 |
| Water | q.s | q.s |

Example 4 is an exemplified Aerosol Gel which is prepared as follows: The water soluble polymers are added to water and mixed until dissolved. The mixture is then heated and the glyceryl oleate, sorbital and fattyacids are added at about 60°C and mixture whilst heating. At 80-85°C triethanolamine is added and mixed for about 20minute to form and aqueous soap phase. After cooling the remaining ingredients including silwet are added at room temperature and mixed well. Water may be added. The mixture is combined with volatile post foaming agent under pressure within a filling line and filled into bottom gassed aerosol cans wth shearing through the valve under nitrogen pressure.

Example 5 is an exemplified Aerosol Foam prepared as follows: Water is heated to 80-85°C after which stearic acid is added and melted. After melting laureth-23 is added and melted and mixed well. Triethanolamine is added and mixed for 30minutes to form a soap. The resulting soap was cooled to about 65°C after which sodium lauryl sulfate is added and mixed BHT is then added and the resulting composition is then cooled to room temperature and the silwet and fragrance are added. The product is packaged with Aero A-31 propellant in an aerosl can using conventional techniques.

| **Ingredient** | **Example 6 (%)** |
|---|---|
| Sodium laureth sulphate (25% w/w active) | 22.0 |
| Glycerin | 3.0 |
| Cocamidopropylbetaine | 2.7 |
| Carbopol Ultrez 20 | 1.5 |
| 10% w/w sodium hydroxide solution | 0.78 |
| Silwet L7210 | 0.5 |
| Phenoxyethanol | 0.5 |
| Benzophenone 4 | 0.14 |
| Disodium EDTA | 0.1 |
| Methylchloroisothizoline and methylisothiazoline (Kathon CG) | 0.03 |
| Water | q.s |

The above example 6 is prepared as follows: Dissolve the benzophenone and sodium hydroxide in an appropriate amount of water to aid manufacture. Add water and glycerin and agitate gently. Then add and carefully disperse the thickener (Carbopol) while stirring (at about 250rpm). Slowly add the sodium laureth sulphate and benzophenone 4 premix. Next add the cocamidopropylbetaine, Kathon CG and Silwet. Add the sodium hydroxide premix and continue to mix until fully homogeneous.

As used herein, molecular weights (mol.wt.s) are provided in unified atomic mass units, daltons, or g/mol.

All parts, ratios, and percentages herein, in the Description, Examples, and Claims, are by weight of the composition and all numerical limits are used with the normal degree of accuracy afforded by the art, unless otherwise specified.

The dimensions and values disclosed herein are not to be understood as being strictly limited to the exact numerical values recited. Instead, unless otherwise specified, each such dimension is intended to mean both the recited value and a functionally equivalent range surrounding that value.

## Claims

1. A composition dispensing hair removal device, said device comprising a composition comprising from 0.1% to 60% by weight of a silicone polyether block copolymer wherein said silicone polyether block copolymer comprises from 1 to 50%, by weight of polyethylene oxide, from 20% to 90% by weight of polypropylene oxide and from 1% to 20%, more preferably 15% by weight of silicone wherein said silicone polyether block copolymer has a molecular weight of from 10000 to 15000.

2. The device according to claim 1, wherein said silicone polyether block copolymer comprises from 1% to 30%, by weight of polyethylene oxide, from 20% to 80% by weight of polypropylene oxide and from 1 to 20%, more preferably 15% by weight of silicone.

3. The device according to any one of the preceding claims, wherein said silicone polyether block copolymer has a ratio of polyethylene oxide to polypropylene oxide of from 2 to 0.1, preferably from 0.6 to 0.25.

4. The device according to claim 1, wherein said composition further comprises from 0.1% to 8% by weight of a water soluble polymer and preferably wherein the ratio of said water soluble polymer to said silicone block copolymer is from 1:5 to 5:1, preferably from 3:1 to 1:3.

5. The device according to claim 4 wherein said water soluble polymer is selected from polyethylene oxide, polyvinyl pyrrolidone, polyacrylamide, polyhydroxymethacrylate, polyvinyl imidazoline, polyethylene glycol, polyvinyl alcohol, polyhydroxyethymethacrylate, guars, celluloses, modified celluloses and mixtures thereof.

6. The device according to claim 4, wherein said water soluble polymer is polyethylene oxide having an average molecular weight of at least 300000, preferably from 300,000 to 8 Million, more preferably from 1 million to 5 million, most preferably 2 to 3 million.

7. The device according to any one of the preceding claims, wherein said composition further comprising from 0.1% to 8%, preferably from 0.1% to 5%, by weight of the lubricating material of a copolymer of polyethylene oxide and polypropylene oxide.

8. The device according to any one of the proceeding claims wherein said composition comprises from 0.1% to 5% of a thickening agent and at least about 30% by weight of water.

9. The device according to any one of the preceding claims, wherein said device comprises a handle connected to a hair removal head, wherein said handle comprises a cavity for housing said composition disposed within said handle and an actuator adapted to displace said composition from said cavity to a fluid dispensing member.

10. The device according to claim 9, wherein said fluid dispensing member comprises an elongated contact region forming at least one dispensing orifice.

11. The device according to claim 10, wherein said elongate elastomeric contact region forms a one way valve, preferably selected from a flap valve, a slit valve, a duckbill valve and a combination thereof.

12. A personal care composition comprising from 40% to 95% by weight of water, 1% to 6% by weight of a volatile post foaming agent and from 0.1% to 60% by weight of silicone polyether block copolymer wherein said silicone polyether block copolymer comprises from 1 to 50%, by weight of polyethylene oxide, from 20% to 90% by weight of polypropylene oxide and from 1 to 20%, more preferably 15% by weight of silicone, wherein said silicone polyether block copolymer has a molecular weight of from 10000 to 15000.

## Patentansprüche

1. Haarentfernungsvorrichtung zum Abgeben einer Zusammensetzung, wobei die Vorrichtung eine Zusammensetzung umfasst, umfassend von 0,1 Gew.-% bis 60 Gew.-% ein Polyether-Blockcopolymer, wobei das Polyether-Blockcopolymer von 1 bis 50 Gew.-% Polyethylenoxid, von 20 Gew.-% bis 90 Gew.-% Polypropylenoxid und von 1 Gew.-% bis 20 Gew.-%, mehr bevorzugt 15 Gew.-% Silikon umfasst, wobei das Silikonpolyether-Blockcopolymer ein Molekulargewicht von 10000 bis 15000 aufweist.

2. Vorrichtung nach Anspruch 1, wobei das Silikonpolyether-Blockcopolymer von 1 Gew.-% bis 30 Gew.-% Polyethylenoxid, von 20 Gew.-% bis 80 Gew.-% Polypropylenoxid und von 1 Gew.-% bis 20 Gew.-%, mehr bevorzugt von 15 Gew.-%, Silikon umfasst.

3. Vorrichtung nach einem der vorstehenden Ansprüche, wobei das Silikonpolyether-Blockcopolymer ein Verhältnis von Polyethylenoxid zu Polypropylenoxid von 2 zu 0,1, vorzugsweise von 0,6 zu 0,25, aufweist.

4. Vorrichtung nach Anspruch 1, wobei die Zusammensetzung ferner von 0,1 Gew.-% bis 8 Gew.-% ein wasserlösliches Polymer umfasst und wobei das Verhältnis des wasserlöslichen Polymers zu dem Silikon-Blockcopolymer von 1:5 bis 5:1, vorzugsweise von 3:1 bis 1:3.

5. Vorrichtung nach Anspruch 4, wobei das wasserlösliche Polymer aus Polyethylenoxid, Polyvinylpyrrolidon, Polyacrylamid, Polyhydroxymethacrylat, Polyvinylimidazolin, Polyethylenglykol, Polyvinylalkohol, Polyhydroxyethymethacrylat, Guaren, Cellulosen, modifizierten Cellulosen und Mischungen davon ausgewählt ist.

6. Vorrichtung nach Anspruch 4, wobei das wasserlösliche Polymer Polyethylenoxid ist, das ein durchschnittliches Molekulargewicht von mindestens 300000, vorzugsweise von 300.000 bis 8 Millionen, mehr bevorzugt von 1 Million bis 5 Millionen, am meisten bevorzugt von 2 bis 3 Millionen aufweist.

7. Vorrichtung nach einem der vorstehenden Ansprüche, wobei die Zusammensetzung ferner von 0,1 Gew.-% bis 8 Gew.-%, vorzugsweise von 0,1 Gew.-% bis 5 Gew.-% das Gleitmaterial eines Copolymers aus Polyethylenoxid und Polypropylenoxid umfasst.

8. Vorrichtung nach einem der vorstehenden Ansprüche, wobei die Zusammensetzung von 0,1 Gew.-% bis 5 Gew.-% ein Verdickungsmittel und mindestens etwa 30 Gew.-% Wasser umfasst.

9. Vorrichtung nach einem der vorstehenden Ansprüche, wobei die Vorrichtung einen Griff umfasst, der mit einem Haarentfernungskopfstück verbunden ist, wobei der Griff einen Hohlraum zum Aufnehmen der Zusammensetzung, die in dem Griff angeordnet ist, und ein Betätigungselement umfasst, das konzipiert ist, die Zusammensetzung von dem Hohlraum zu einem Fluidabgabeelement zu verschieben.

10. Vorrichtung nach Anspruch 9, wobei das Fluidabgabeelement einen verlängerten Kontaktbereich umfasst, der mindestens eine Abgabeöffnung bildet.

11. Vorrichtung nach Anspruch 10, wobei der verlängerte Elastomerkontaktbereich ein Einwegventil bildet, das vorzugsweise aus einem Klappenventil, einem Schlitzventil, einem Entenschnabelventil und einer Kombination davon ausgewählt ist.

12. Körperpflegezusammensetzung, umfassend von 40 Gew.-% bis 95 Gew.-% Wasser, von 1 Gew.-% bis 6 Gew.-% ein flüchtiges nachschäumendes Mittel und von 0,1 Gew.-% bis 60 Gew.-% ein Polyether-Blockcopolymer, wobei das Polyether-Blockcopolymer von 1 bis 50 Gew.-% Polyethylenoxid, von 20 Gew.-% bis 90 Gew.-% Polypropylenoxid und von 1 Gew.-% bis 20 Gew.-%, mehr bevorzugt 15 Gew.-% Silikon umfasst, wobei das Silikonpolyether-Blockcopolymer ein Molekulargewicht von 10000 bis 15000 aufweist.

## Revendications

1. Dispositif d'épilation à distribution de composition, ledit dispositif comprenant une composition comprenant de 0,1 % à 60 % en poids d'un copolymère séquencé de polyéther de silicone dans lequel ledit copolymère séquencé de polyéther de silicone comprend de 1 à 50 %, en poids d'oxyde de polyéthylène, de 20 % à 90 % en poids d'oxyde de polypropylène et de 1 % à 20 %, plus préférablement 15 % en poids de silicone dans lequel ledit copolymère séquencé de polyéther de silicone a une masse moléculaire allant de 10 000 à 15 000.

2. Dispositif selon la revendication 1, dans lequel ledit copolymère séquencé de polyéther de silicone comprend de 1 % à 30 %, en poids d'oxyde de polyéthylène, de 20 % à 80 % en poids d'oxyde de polypropylène et de 1 à 20 %, plus préférablement 15 % en poids de silicone.

3. Dispositif selon l'une quelconque des revendications précédentes, dans lequel ledit copolymère séquencé de polyéther de silicone a un rapport de l'oxyde de polyéthylène à l'oxyde de polypropylène allant de 2 à 0,1, de préférence de 0,6 à 0,25.

4. Dispositif selon la revendication 1, dans lequel ladite composition comprend en outre de 0,1 % à 8 % en poids d'un polymère hydrosoluble et de préférence dans lequel le rapport dudit polymère hydrosoluble audit copolymère séquencé de silicone va de 1:5 à 5:1, de préférence de 3:1 à 1:3.

5. Dispositif selon la revendication 4 dans lequel ledit polymère hydrosoluble est choisi parmi oxyde de polyéthylène, polyvinylpyrrolidone, polyacrylamide, polyhydroxyméthacrylate, polyvinyl-imidazoline, polyéthylène glycol, alcool polyvinylique, polyhydroxyéthyméthacrylate, gommes de guar, celluloses, celluloses modifiées et des mélanges de ceux-ci.

6. Dispositif selon la revendication 4, dans lequel ledit polymère hydrosoluble est de l'oxyde de polyéthylène ayant une masse moléculaire moyenne d'au moins 300 000, de préférence de 300 000 à 8 millions, plus préférablement de 1 million à 5 millions, le plus préférablement 2 à 3 millions.

7. Dispositif selon l'une quelconque des revendications précédentes, dans lequel ladite composition comprend en outre de 0,1 % à 8 %, de préférence de 0,1 % à 5 %, en poids du matériau lubrifiant d'un copolymère d'oxyde de polyéthylène et d'oxyde de polypropylène.

8. Dispositif selon l'une quelconque des revendications précédentes dans lequel ladite composition comprend de 0,1 % à 5 % d'un agent épaississant et au moins environ 30 % en poids d'eau.

9. Dispositif selon l'une quelconque des revendications précédentes, dans lequel ledit dispositif comprend une poignée reliée à une tête d'épilation, dans lequel ladite poignée comprend une cavité destinée à loger ladite composition disposée au sein de ladite poignée et un actionneur conçu pour déplacer ladite composition de ladite cavité vers un élément de distribution de fluide.

10. Dispositif selon la revendication 9, dans lequel ledit élément de distribution de fluide comprend une région de contact allongée formant au moins un orifice de distribution.

11. Dispositif selon la revendication 10, dans lequel ladite région de contact élastomère allongée forme un clapet antiretour, choisi de préférence parmi une soupape à clapet, une soupape fendue, une soupape à bec-de-canard et une combinaison de celles-ci.

12. Composition de soins personnels comprenant de 40 % à 95 % en poids d'eau, 1 % à 6 % en poids d'un agent post-moussant volatil et de 0,1 % à 60 % en poids d'un copolymère séquencé de polyéther de silicone dans laquelle ledit copolymère séquencé de polyéther de silicone comprend de 1 à 50 %, en poids d'oxyde de polyéthylène, de 20 % à 90 % en poids d'oxyde de polypropylène et de 1 à 20 %, plus préférablement 15 % en poids de silicone, dans laquelle ledit copolymère séquencé de polyéther de silicone a une masse moléculaire allant de 10 000 à 15 000.
